# EUROPÄISCHE PATENTANMELDUNG

(11) **EP 0 571 787 A1**
(43) Veröffentlichungstag der Anmeldung: **01.12.1993**
(21) Anmeldenummer: 93107191.4
(22) Anmeldetag: 04.05.1993
(51) Int. Cl.: A61L 29/00, A61L 33/00

(54) **Medizinische Arbeitsmittel**

(30) Priorität: 23.05.1992 DE 4217165
(71) Anmelder: REHAU AG + Co, 95111 Rehau (DE)
(72) Erfinder: Kühlein, Georg, W-8689 Röslau (DE)

(57) **Zusammenfassung**

Die Erfindung betrifft medizinische Arbeitsmittel wie Katheter, Schläuche, Behälter, Formteile und dergleichen aus polymeren Materialien. Diese Arbeitsmittel besitzen eine besondere Ausstattung zur Erhöhung der Blutkompatibilität bei der Berührung mit Blut oder blutähnlichen Flüssigkeiten. Das Kennzeichnende der Erfindung wird darin gesehen, daß in das Ausgangspolymer vor der eigentlichen Formgebung eine definierte Menge von hydroxiorganofunktionellen Polycaprolacton-Polydimethylsiloxan-Copolymeren der Formel 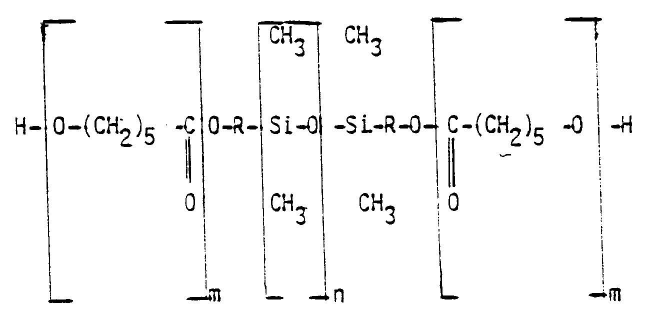
als Zuschlagstoffe eingemischt sind.

## Beschreibung

Die Erfindung betrifft medizinische Arbeitsmittel wie Katheter, Schläuche, Behälter, Formteile und dergleichen aus polymeren Materialien, wobei diese Arbeitsmittel eine besondere Ausstattung zur Erhöhung der Blutkompatibilität bei der Berührung mit Blut oder blutähnlichen Flüssigkeiten aufweisen.

Aus der DE-A 19 30 136 ist ein beschichteter Katheter bekannt, welcher als Basis aus einem Schlauch aus Gummi oder einem Elastomeren besteht. Dieser Katheterschlauch ist an der Außen- und/oder Innenwand mit einem hydrophilen Polyacrylat bzw. -Methacrylat, insbesondere mit einem Hydroxialkyl- oder einem Hydroxialkoxi-Alkyl-Acrylat bzw. Methacrylat mit jeweils niederen Alkylresten beschichtet.

Weiterhin ist es bekannt, derartige medizinische Arbeitsmittel mit einer Beschichtung aus Heparin zu versehen. Diese Heparin-Beschichtungsverfahren arbeiten über APTES- und TDMAC-Brücken. Mit dieser Heparin-Beschichtung wird das Anhaften von Blutplättchen an der Oberfläche des medizinischen Arbeitsmittels verhindert und gleichzeitig die intrinsische Koagulation desaktiviert. Ausführungen zum Beschichtungsverfahren mit Heparin über TDMAC-Brücken finden sich bei G.A. Grode, R.D. Falb, J.P. Crowley in J. Biomed. Mater. Res. Symp., 3, 77 (1972). Entsprechende Hinweise zur Beschichtung mit Heparin über APTES-Brücken finden sich bei R.L. Merker, L.J. Elyasch, S.W. Mayhew, J.Y.C. Wang in Proc. Artif. Heart Conf., 1969, Seite 29.

Alle diese Verfahren haben Gen prinzipiellen Nachteil, daß sie technisch aufwendung und sehr arbeitsintensiv sind. So läuft eine Heparinisierung bei beiden genannten Verfahren in mehreren zusätzlichen Arbeits- und Beschichtungszyklen ab.

Ein weiterer wesentlicher Nachteil dieser bekannten Verfahren ist, daß die auf diese Weise hergestellten medizinischen Halbzeuge sterilisiert werden müssen, weil die Beschichtung mit Heparin mikrobiell abgebaut werden kann. Da nach der Konfektion der Halbzeuge zu Fertigprodukten eine Gesamtsterilisation erforderlich ist, ist hier als weiterer Nachteil das erhebliche Risiko einer Mehrfachsterilisation zu nennen.

Hier setzt die Erfindung ein, die es sich zur Aufgabe gestellt hat, die zum Stand der Technik genannten Nachteile zu vermeiden und eine Methode zur Erzielung der optimalen Blutkompatibilität bei medizinischen Halbzeugen aufzuzeigen, bei der über das medizinische Arbeitsmittel weder das biologische System negativen Auswirkungen ausgesetzt ist noch das Material, aus dem das medizinische Arbeitsmittel gefertigt ist durch Einwirkung des biologischen Systems so weit geschädigt werden kann, daß es die vorgesehene Funktion nicht mehr erfüllt. Erfindungsgemäß wird dazu vorgeschlagen, daß in das Ausgangspolymere vor der eigentlichen Formgebung eine definierte Menge von hydroxiorganofunktionellen Polycaprolacton-Polydimethylsiloxan-Copolymere der Formel 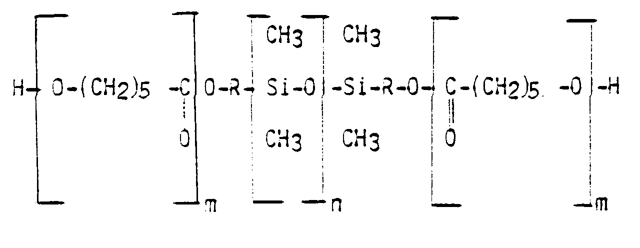
als Zuschlagstoffe eingemischt sind.

Die Erfindung geht von der Überlegung aus, dem Ausgangspolymeren Additive zuzusetzen, die den Werkstoff von der Blutkompatibilität her mit seinem relativen Gerinnungsparameter dem Quotienten 1,0 annähern.

Dieses Vorhaben ist durch die erfindungsgemäße Addition von hydroxiorganofunktionellen Polycaprolacton-Polydimethylsiloxan-Copolymeren gelungen. SO hat es sich bei der Verwendung von weichmacherhaltigem Polyvinylchlorid als Ausgangsmaterial für die Herstellung medizinischer Arbeitsmittel als vorteilhaft herausgestellt, daß die heute für solche Anwendungszwecke üblichen PVC-Rezepturen auf Basis Zink und Kalzium erfindungsgemäß durch die genannten Copolymere ergänzt werden. Hierbei hat sich als zweckmäßig eine Dosierungsrate von 0,01 bis 2,0 Gewichtsprozent erwiesen.

Bei der Verwendung anderer Polymere zur Herstellung der medizinischen Arbeitsmittel können die erfindungsgemäßen Copolymere die eingesetzten Arbeitshilfen wie Gleitmittel und dergleichen ergänzen oder ersetzen. Hier haben sich Anteile zwischen 0,02 und 1,5 Gewichtsprozent als vorteilhaft erwiesen.

Die mit den erfindungsgernäßen Copolymeren ausgestatteten medizinischen Arbeitsmittel verhindern die Blutplättchen-Aggregation und desaktivieren die intrinsische Koagulation. Eine Vorsterilisierung der Halbzeuge ist nicht mehr erforderlich, da die erfindungsgemäßen Copolymere entgegen der bisher üblichen Heparinbeschichtung nicht mehr mikrobiell abgebaut werden können.

Die nachfolgenden Beispiele zeigen die Verwendungsmöglichkeit der erfindungsgemäßen Copolymere als Zuschlagstoffe für polymere Materialien zur Herstellung medizinischer Arbeitsmittel.

### Beispiel 1:

### Weich-PVC-Rezeptur

100 Teile S-PVC
10-90 Teile organischen Weichmacher
1-10 Teile epoxidierte Öle
0,1-1,0 Teile Metallseifen
0,1-0,6 Teile Gleitmittel
0,01-2 Teile hydroxiorganofunktionelle Polycaprolacton-Polydimethylsiloxan-Copolymere der allgemeinen Formel: 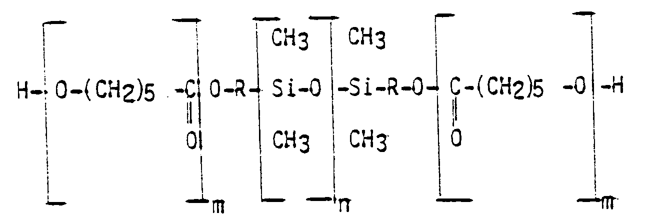
Die in der Formel verwendeten Buchstaben m, n bezeichnen die Anteile der jeweils gekennzeichneten Formelbereiche. Erfindungsgemäß liegen diese Anteile bei
m - zwischen 5 und 35, bei
n - zwischen 10 und 60 Teilen.

### Beispiel 2:

### Polyurethan-Rezeptur

100 Teile Polyurethan
0,02-1,5 Teile hydroxiorganofunktionelles Polycaprolacton-Polydimethyisiloxan-Copolymer der Formel gemäß Beispiel 1.

### Beispiel 3:

### Polyethylen-Rezeptur

100 Teile Polyethylen
0,01-1,7 Teile hydroxiorganofunktionelles Polycaprolacton-Polydimethylsiloxan-Copolymer der Formel gemäß Beispiel 1.

### Beispiel 4:

### Polypropylen-Rezeptur

100 Teile Polypropylen
1,0-3 Teile Metallseifen
0,01-3 Teile hydroxiorganofunktionelles Polycaprolacton-Polydimethylsiloxan-Copolymer der Formel gemäß Beispiel 1.

## Patentansprüche

1. Medizinische Arbeitsmittel wie Katheter, Schläuche, Behälter, Formteile und dergleichen aus polymeren Materialien, wobei diese Arbeitsmittel eine besondere Ausstattung zur Erhöhung der Blutkompatibilität bei der Berührung mit Blut oder blutähnlichen Flüssigkeiten aufweisen, dadurch gekennzeichnet, daß in das Ausgangspolymere vor der eigentlichen Formgebung eine definierte Menge von hydroxiorganofunktionellen Polycaprolacton-Polydimethylsiloxan-Copolymeren der Formel 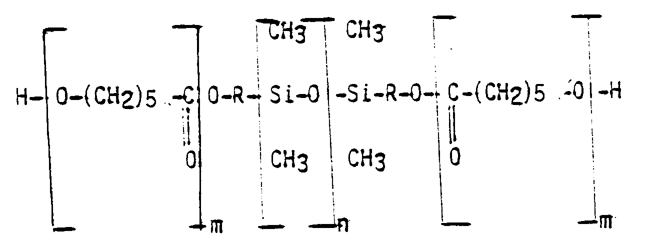 als-Zuschlagstoffe eingemischt sind.

2. Medizinische Arbeitsmittel nach Anspruch 1, dadurch gekennzeichnet, daß die Menge der hydroxiorganofunktionellen Polycaprolacton-Polydimethylsiloxan-Copolymere zwischen 0,01 und 3,0 Gewichtsprozent liegt.
